Europäisches Patentamt

European Patent Office

Office européen des brevets

(11) Publication number: **0 069 776**
**B1**

# (12) EUROPEAN PATENT SPECIFICATION

(45) Date of publication of patent specification: **16.04.86**

(51) Int. Cl.⁴: **G 02 B 6/44, A 61 N 5/06**

(21) Application number: **82900717.8**

(22) Date of filing: **15.01.82**

(86) International application number:
**PCT/US82/00048**

(87) International publication number:
**WO 82/02604 05.08.82 Gazette 82/19**

(54) **An optical fiber assembly and method of mounting it.**

(30) Priority: **21.01.81 US 226669**

(43) Date of publication of application:
**19.01.83 Bulletin 83/03**

(45) Publication of the grant of the patent:
**16.04.86 Bulletin 86/16**

(84) Designated Contracting States:
**DE FR GB NL**

(56) References cited:
**EP-A-0 025 969**
**GB-A-1 234 958**
**GB-A-2 028 530**
**US-A-2 235 979**
**US-A-3 610 231**
**US-A-3 756 688**
**US-A-3 809 072**
**US-A-4 170 997**
**US-A-4 266 534**
**US-A-4 266 549**
**US-A-4 273 109**
**PATENT ABSTRACTS OF JAPAN, vol. 2, no. 40,**
**March 16, 1978, page 271 E 78**

(73) Proprietor: **Hughes Aircraft Company**
**Centinela & Teale Streets**
**Culver City California 90230 (US)**

(72) Inventor: **MYER, Jon H.**
**22931 Gershwin Drive**
**Woodland Hills, CA 91364 (US)**

(74) Representative: **Milhench, Howard Leslie et al**
**A.A. Thornton & Co. Northumberland House**
**303/306 High Holborn**
**London, WC1V 7LE (GB)**

Courier Press, Leamington Spa, England.

## Description

This invention is directed to an optical fiber assembly for use in a biological environment, as well as to a method of mounting said assembly.

U.S. Patent 4,170,997 to Douglas A. Pinnow and Anthony L. Gentile is directed to a medical laser instrument which employs a flexible fiber optical endoscope which is suitable for application of long wavelength, high power laser radiation for surgical purposes inside the human body. To accomplish this purpose, the optical fiber has a sheath which prevents contact between the body fluid and the fiber optic waveguide material which is capable of infrared transmission.

When non-refractory optical fibers are used to transmit long wave infrared radiation, it is often necessary to protect them from a hostile environment. For example, fibers of alkali halides, while excellent transmitters of infrared radiation, are very soluble in water and easily corroded by moisture. It is thus necessary to protect such fibers against body fluids. Alternate fiber materials such as thallium halide compounds are toxic, and thus the body and the remainder of the exterior environment must be protected from exposure to the noxious fiber material. In either case, it becomes necessary to surround the fiber with a hermetic protective sheath while providing a means for the radiation to enter or emerge at the end of the fiber.

Thus, US—A—4170997 discloses an optical fiber assembly comprising an elongated physiologically toxic optical fiber having ends and being at least partially transparent to radiation in the infra-red wavelength band, a physiologically non-toxic sheath surrounding said optical fiber adjacent at least one end thereof, said sheath being tubular with a bore therein extending beyond the end of the optical fiber, and a window within said sheet adjacent said end of said optical fiber, said window being physiologically non-toxic and at least partially transparent to radiation in the same infra-red wavelength band to which said optical fiber is at least partially transparent, said window being mounted within said sheet in the manner such as to hermetically seal and plug the end thereof.

However the problem with such an arrangement is that it is rather bulky and non-flexible. It is an object of the present invention to provide an optical fiber assembly which is compact and relatively flexible so that it can be used in a variety of situations. The object of the present invention is solved briefly by inserting into the end of the sheath surrounding the optical fiber a window formed as a solid plug and employing the sheath to hold the window in position.

More specifically in one aspect the present invention provides an optical fiber assembly for use in a biological environment comprising:

an elongated optical fiber having ends, said fiber being at least partially transparent to radiation in the infra-red wavelength band;

a physiologically non-toxic sheath surrounding said optical fiber adjacent at least one end thereof, said sheath being tubular with a bore therein extending beyond the end of said optical fiber;

a window within said sheath adjacent said end of said optical fiber to hermetically seal and plug the end of said tubular sheath, said window being physiologically non-toxic, said window being at least partially transparent to radiation in the infrared wavelength band of the same wavelength to which said optical fiber is at least partially transparent;

characterized in that said sheath is made of malleable material, said sheath bore has a swaged divergent interior surface adjacent the end of said sheath and said window is in the form of a plug, the lateral surface of the plug comprising a convergent swaging exterior surface resiliently engaged against and within said divergent swaged interior surface of said sheath bore and said sheath resiliently embracing and engaging said window.

In a further aspect the invention provides a method of attaching a non-toxic window plug within the end of a non-toxic malleable tubular sheath having an optical fiber therein, the window plug and the optical fiber both being at least partially transparent to radiation in the infra-red wavelength band of the same wavelength, and the sheath extending beyond the end of the optical fiber, said method comprising the steps of:

forming the window plug with inside and outside window end-surfaces thereon with respect to the optical fiber and with a lateral convergent surface thereon so that the window end-surface adjacent the smaller end of the convergent surface is the inside window end of the convergent surface is the inside window end-surface;

positioning the window plug with its inside window end-surface directed toward the open end of the malleable tubular sheath; and

pressing the plug with its inside window end-surface first into the open bore of the tubular sheath so that the divergent surface on the window malleably outwardly deforms the sheath to produce a resilient hermetic sealing engagement of the sheath onto the window.

Brief Description of the Drawings

FIG. 1 is a side elevational view of an optical fiber sheath with a miniature window plug therein in accordance with this invention, with parts broken away and parts taken in central section.

FIG. 2 is a side elevational view of the structure before insertion of the window plug into the end of the bore of the optical fiber sheath.

FIG. 3 is a side elevational view, with parts broken away and parts taken in section of the end of the fiber optic sheath, after insertion of the miniature window therein and before final sealing.

Description of the Preferred Embodiment

In the preferred embodiment, optical fiber 10 is substantially transmissive to long wave infrared radiation, and many such fibers are soluble in water, easily corroded by moisture or are toxic. Examples of suitable alkali halide fibers which are soluble include sodium chloride, potassium chloride and potassium iodide. Examples of toxic fibers are thallium halides, including thallium bromoiodide also known as KRS-5 and thallium bromide. In order to protect the optical fiber 10 against its environment and to protect the environment against the optical fiber 10, sheath 12 is provided.

Sheath 12 is a malleable tubular metallic sheath, for example, a 24 gauge stainless steel or platinum syringe needle tube. Optical fiber 10 is placed therein, with its end 14 recessed with respect to the end 16 of the sheath.

The external surface of sheath 12 is tapered down toward the end 16 to form a right conical surface 18 about the central axis 20 of the entire structure. The conical shaping of the end of the sheath reduces the wall thickness but stops short of forming a knife edge at end 16.

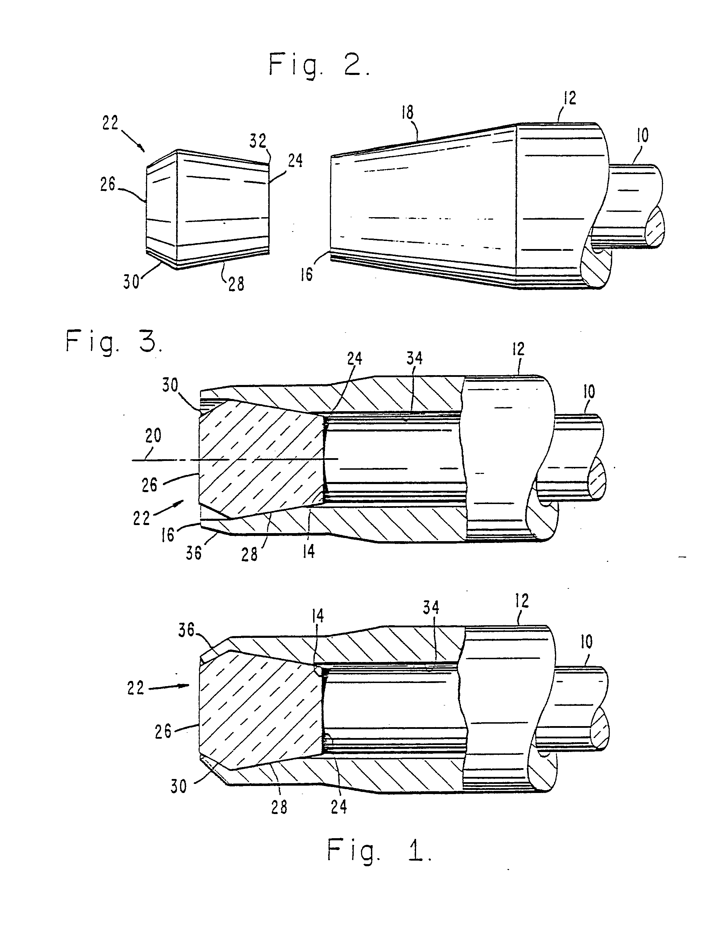

Window 22 is a structure which is transparent to the wavelength of interest. For infrared radiation, window 22 can be made of diamond, germanium, zinc selenide or silicon. Window 22 is also a body of revolution about the axis 20. It has inner and outer planar ends 24 and 26, both normal to the axis 20. The outer lateral surface of window 22 is comprised of a first right conical surface 28 and a second right conical surface 30. Both of these right conical surfaces are surfaces of revolution about axis 20. The first conical surface 28 is at the same conical angle as the original conical surface 18 on the exterior of sheath 12, as seen in FIG. 2. The truncated diameter of conical surface 28 adjacent the inner planar end 24 is the same as the diameter of the bore 34 of sheath 12.

As is seen in FIGS. 2 and 3, window 22 is first placed adjacent the open end of sheath 12 and then the conical surface 28 is forcibly inserted into the open end of sheath 12. Thrust of the window 22 into the malleable sheath 12 causes expanding deformation of the sheath so that when the window is fully inserted as is illustrated in FIG. 3, the entire conical surface 28 is in hermetic sealing contact with the sheath. The residual deformation stress causes continued compression of the sheath onto the window on conical surface 28.

In order to retain window 22 in place, the end portion or lip 36 of the sheath extending over the surface 30 is swaged down onto the surface 30. With the window deforming the bore of the sheath, a hermetic seal is formed, and the window is locked in place by the inwardly swaged end portion of lip 36.

As an aid toward pressing window 22 into the bore, the total included angle of conical surface 28 was chosen to be 20°, but alternate angles can be employed, as required by the malleability characteristics of sheath 12.

As stated above, suitable materials for window 22 include diamond, germanium and zinc selenide and silicon. These materials have essentially no solubility in fluids with which they might come in contact in a human body. All these materials have good transparency in the long wavelength part of the infrared spectrum. All these materials have a large refractive index and thus it is advantageous to provide the window 22 with an anti-reflection coating to maximize transmission. The coating on facet 24 may be any standard coating. However, the coating on facet 26 is exposed to body fluids and must be inert. A multi-lever coating such as barium fluoride-zinc selenide will be reasonably resistance to such exposure.

## Claims

1. An optical fiber assembly for use in a biological environment comprising:
an elongated optical fiber (10) having ends, said fiber being at least partially transparent to radiation in the infra-red wavelength band;
a physiologically non-toxic sheath (12) surrounding said optical fiber (10) adjacent at least one end thereof, said sheath being tubular with a bore (34) therein extending beyond the end of said optical fiber;
a window (22) within said sheath (12) adjacent said end of said optical fiber (10) to hermetically seal and plug the end of said tubular sheath, said window being physiologically non-toxic, said window being at least partially transparent to radiation in the infra-red wavelength band of the same wavelength to which said optical fiber is at least partially transparent;
characterized in that said sheath is made of malleable material, said sheath bore has a swaged divergent interior surface adjacent the end of said sheath, and said window is in the form of a plug, the lateral surface of the plug comprising a convergent swaging exterior surface (28) resiliently engaged against and within said divergent swaged interior surface of said sheath bore, and said sheath (12) resiliently embracing and engaging said window (22).

2. The fiber optical assembly of claim 1 wherein said divergent bore of said sheath and said convergent surface (28) of said window are conical surfaces of revolution about an axis centrally positioned through said optical fiber (10) and said sheath (12).

3. The optical fiber assembly of claim 2 wherein said window (22) and said fiber (10) are at least partially transparent to infra-red radiation in the long wavelength infra-red band ranging from 8 to 30 micrometers wavelength.

4. The fiber optical assembly of claim 1 wherein the end of said sheath extends beyond the convergent external surface of said window to form lip (36) and said lip (36) is positioned against said window (22) beyond said convergent surface (28) for retaining said window within said sheath and for retaining said convergent surface (28) of said window against said divergent bore surface within said sheath.

5. The optical fiber assembly of claim 1 including a lateral retaining surface (30) on said window extending away from said convergent swaging exterior surface (28) in the direction away from said optical fiber (10), said sheath (12) engaging on said retaining surface (30) on said window (22) to retain said window within said sheath and retain said convergent swaging surface (28) of said window against said swaged divergent bore surface within said sheath.

6. The fiber optical assembly of claim 5 wherein said convergent surface (28) on said window is conical and said retaining surface (30) on said window is a divergent conical surface thereon, said sheath engaging on said divergent surface (30) to retain said window within said sheath.

7. The fiber optical assembly of claim 6 wherein said convergent surface (28) on said window is a truncated right circular cone.

8. A method of attaching a non-toxic window plug (22) within the end of a non-toxic malleable tubular sheath (12) having an optical fiber (10) therein, the window plug and the optical fiber both being at least partially transparent to radiation in the infra-red wavelength band of the same wavelength, and the sheath extending beyond the end of the optical fiber, said method comprising the steps of:

forming the window plug (22) with inside and outside window end-surfaces (24, 26) thereon with respect to the optical fiber and with a lateral convergent surface (28) thereon so that the window end-surface adjacent the smaller end of the convergent surface is the inside window end-surface;

positioning the window plug (22) with its inside window end-surface (24) directed toward the open end of the malleable tubular sheath; and

pressing the plug with its inside window end-surface first into the open bore of the tubular sheath so that the convergent surface (28) on the window malleably outwardly deforms the end of the sheath to produce a resilient hermetic sealing engagement of the sheath onto the window.

9. The method of claim 8 further including the step of plastically deforming an overhanging lip (36) of said tubular sheath (12) around the periphery of the window plug (22) outwardly of said convergent surface to retain and lock the window within the sheath.

**Revendications**

1. Dispositif à fibre optique pour utilisation en environnement biologique comprenant:

— une fibre optique allongée (10), munie d'extrémités, cette fibre étant au moins partiellement transparente aux radiations dans la bande des longueurs d'ondes infra-rouges;

— une gaine physiologiquement non toxique (12), entourant la fibre optique (10) au voisinage de l'une au moins de ses extrémités, cette gaine étant tubulaire et présentant un alésage intérieur (34) s'étendant au-delà de l'extrémité de la fibre optique;

— dans la gaine (12), une fenêtre (22) adjacente à ladite extrémité de la fibre optique (10) de manière à sceller et boucher hermétiquement l'extrémité de la gaine tubulaire, cette fenêtre étant physiologiquement non toxique, et étant au moins partiellement transparente aux radiations, de la bande des longueurs d'ondes infra-rouges, de même longueur d'onde que celles auxquelles la fibre optique est au moins partiellement transparente;

caractérisé en ce que la gaine est faite en matière malléable, en ce que l'alésage de la gaine a au voisinage de l'extrémité de la gaine une surface intérieure divergente déformée, en ce que la fenêtre est en forme de bouchon, la surface latérale du bouchon comprenant, une surface extérieure déformatrice convergente (28) élastiquement enfoncée dans et contre la surface intérieure divergente déformée de l'alésage de la gaine, et en ce que la gaine entoure la fenêtre (22) élastiquement et avec serrage.

2. Dispositif à fibre optique selon la revendication 1 dans lequel l'alésage divergent de la gaine et la surface convergente (28) de la fenêtre sont des surfaces coniques de révolution autour d'un axe situé au centre de la fibre optique (10) et de la gaine (12).

3. Dispositif à fibre optique selon la revendication 2 dans lequel la fenêtre (22) et la fibre (10) sont au moins partiellement transparentes aux radiations infra-rouges dans la bande des grandes longueurs d'ondes infra-rouges s'étendant de 8 à 30 microns.

4. Dispositif à fibre optique selon la revendication 1 dans lequel l'extrémité de la gaine s'étend au-delà de la surface externe convergente de la fenêtre pour former une lèvre (36), et dans lequel la lèvre (36) est mise en place contre la fenêtre (22) au-delà de la surface convergente (28) pour maintenir la fenêtre dans la gaine et pour maintenir la surface convergente (28) de la fenêtre contre la surface divergente de l'alésage à l'intérieur de la gaine.

5. Dispositif à fibre optique selon la revendication 1 comprenant sur la fenêtre une surface latérale de maintien (30) s'étendant en direction opposée à la fibre optique (10) à partir de la surface extérieure déformatrice convergente (28), la gaine (12) étant en prise avec la surface de maintien (30) de la fenêtre (22) pour maintenir la fenêtre à l'intérieur de la gaine et maintenir la surface déformatrice convergente (28) de la fenêtre contre la surface divergente déformée de l'alésage à l'intérieur de la gaine.

6. Dispositif à fibre optique selon la revendication 5 dans lequel la surface convergente (28) de la fenêtre est conique et la surface de maintien (30) sur cette fenêtre est une surface divergente conique, la gaine étant en prise avec cette surface divergente (30) pour maintenir la fenêtre à l'intérieur de la gaine.

7. Dispositif à fibre optique selon la revendication 6 dans lequel la surface convergente (28) de la fenêtre est un tronc de cône circulaire droit.

8. Procédé pour fixer un bouchon fenêtre non toxique (22) dans l'extrémité d'une gaine tubulaire malléable non toxique (12) contenant une fibre optique (10), le bouchon fenêtre et la fibre optique étant tous deux au moins partiellement transparents à des radiations de mêmes longueurs d'ondes dans la bande des longueurs d'ondes infra-rouges, tandis que la gaine s'étend au-delà de l'extrémité de la fibre optique, ce procédé comprenant les étapes suivantes:

— on forme le bouchon fenêtre (22) avec des surfaces d'extrémité de fenêtre (24, 26), intérieure et extérieure par rapport à la fibre optique, et une surface latérale convergente (28), de sorte que la surface d'extrémité de fenêtre adjacente à la petite extrémité de la surface convergente soit la surface d'extrémité intérieure de la fenêtre;

— on présente le bouchon fenêtre (22) en tournant sa surface d'extrémité intérieure (24) vers l'extrémité ouverte de la gaine tubulaire malléable; et

— on presse le bouchon, avec sa surface d'extrémité intérieure en tête, dans l'alésage ouvert de la gaine tubulaire, de sorte que la surface convergente (28) de la fenêtre déforme malléablement vers l'extérieur l'extrémité de la gaine pour former un scellement hermétique par serrage élastique de la gaine sur la fenêtre.

9. Procédé selon la revendication 8, comprenant en outre l'étape consistant à déformer plastiquement une lèvre dépassante (36) de la gaine tubulaire (12) autour de la périphérie du bouchon fenêtre (22), à l'extérieur de ladite surface convergente pour maintenir et verrouiller la fenêtre à l'intérieur de la gaine.

## Patentansprüche

1. Optische Faseranordnung zur Verwendung in einer biologischen Umgebung mit einer langgestreckten optischen Faser (10), welche Endbereiche aufweist, wobei die Faser wenigstens teilweise für Strahlung in dem Infrarot-Wellenlängenband durchlässig ist; mit einer physiologisch unbedenklichen Ummantelung (12), welche die optische Faser (10) im Nahbereich wenigstens eines der Endbereiche umfaßt, wobei die Ummantelung rohrförmig mit einer Durchgangsöffnung (34) ausgebildet ist, und sich über den Endbereich der optischen Faser hinauserstreckt; mit einem Fenster (22) innerhalb der Ummantelung (12) nahe des einen Endbereiches der optischen Faser (10), um das Ende der rohrförmigen Ummantelung hermetisch zu versiegeln und abzuschließen, wobei das Fenster physiogisch unbedenklich ist und wenigstens teilweise für eine Strahlung in dem infraroten Wellenlängenband der gleichen Wellenlänge durchlässig ist, für das auch die optische Faser wenigstens teilweise durchlässig ist, dadurch gekennzeichnet,

daß die Ummantelung aus einem kaltverformbaren Material ist; daß die Durchgangsöffnung in der Ummantelung nahe des Endes der Ummantelung eine gesenkte divergente Innenoberfläche aufweist; und daß das Fenster in Form eines Pfropfens ausgebildet ist, wobei die laterale Oberfläche des Pfropfens eine konvergente senkende äußere Oberfläche (28) aufweist, welche elastisch gegen und innerhalb der divergenten gesenkten Innenoberfläche der Durchgangsöffnung anliegt, wobei die Ummantelung (12) das Fenster (22) elastisch umfaßt und hält.

2. Faseranordnung nach Anspruch 1, dadurch gekennzeichnet, daß die divergente Bohrung in der Ummantelung und die konvergente Oberfläche (28) des Fensters konische Rotationsflächen bezüglich einer Achse sind, welche mittig durch die optische Faser (10) und die Ummantelung (12) verläuft.

3. Faseranordnung nach Anspruch 2, dadurch gekennzeichnet, daß das Fenster (22) und die Faser (10) wenigstens teilweise für eine Infrarotstrahlung in dem langwelligen Infratrotband zwischen 8 und 30 Mikrometer Wellenlänge durchlässig sind.

4. Faseranordnung nach Anspruch 1, dadurch gekennzeichnet, daß das Ende der Ummantelung sich über die konvergente äußere Oberfläche des Fensters erstreckt, und eine Lippe (36) bildet, wobei die Lippe (36) über die konvergente Oberfläche (28) hinaus gegen das Fenster (22) gelegt wird, um das Fenster innerhalb der Ummantelung zu halten und um die konvergente Oberfläche (28) des Fensters gegen die divergente Oberfläche innerhalb der Ummantelung zu halten.

5. Faseranordnung nach Anspruch 1, dadurch gekennzeichnet, daß eine laterale Haltefläche (30) an dem Fenster ausgebildet ist, welche sich von der konvergenten äußeren Oberfläche (28) in eine Richtung von der optischen Faser (10) wegerstreckt, wobei die Ummantelung (12) mit der Haltefläche (30) an dem Fenster (22) in Anlage gerät, um das Fenster innerhalb der Ummantelung zu halten und um die konvergente Oberfläche (28) des Fensters gegen die divergente Oberfläche innerhalb der Ummantelung zu halten.

6. Faseranordnung nach Anspruch 5, dadurch gekennzeichnet, daß die konvergente Oberfläche (28) an dem Fenster konisch ist und die Haltefläche (30) an dem Fenster als divergente konische Oberfläche ausgebildet ist, wobei die Ummantelung auf der divergenten Oberfläche (30) zu liegen kommt, um das Fenster innerhalb der Ummantelung zu halten.

7. Faseranordnung nach Anspruch 6, dadurch gekennzeichnet, daß die konvergente Oberfläche (28) an dem Fenster ein gerader Kreiskegelstumpf ist.

8. Verfahren zum Anordnen eines nichtgiftigen Fenster-Pfropfens (22) an dem Ende einer nichtgiftigen kalt-verformbaren rohrförmigen Ummantelung (12), welche eine optische Faser (10) in sich aufweist, wobei sowohl der Fenster-Pfropfen als auch die optische Faser wenigstens teilweise für eine Strahlung in dem Infrarot-Wellenlängenband der gleichen Wellenlänge durchlässig sind und die Ummantelung sich über den Endbereich der optischen Faser hinauserstreckt, gekennziechnet durch die folgenden Verfahrensschritte:

Ausbilden des Fenster-Pfropfens (22) mit bezüglich der optischen Faser inneren und äußeren Fenster-Endoberflächen (24, 26) und mit einer lateralen konvergenten Oberfläche (28), so daß die Fenster-Endoberfläche im Bereich des kleineren Endes der konvergenten Oberfläche die innere Fenster-Endoberfläche ist;

Anordnen des Fenster-Pfropfens (22) mit seiner inneren Fenster-Endoberfläche (24) in Richtung auf das offene Ende der kaltverformbaren rohrförmigen Ummantelung; und

Pressen des Pfropfens mit seiner inneren Fenster-Endoberfläche in die offene Ausnehmung der rohrförmigen Ummantelung, so daß die konvergente Oberfläche (28) an dem Fenster den Endbereich der Ummantelung gesenkartig nach außen deformiert, um eine elastische hermetische Versiegelungs-Anlage der Ummantelung an dem Fenster zu erzeugen.

9. Verfahren nach Anspruch 8, dadurch gekennzeichnet, daß eine überhängende Lippe (36) der rohrförmigen Ummantelung (12) um die Umfangsoberfläche des Fenster-Pfropfens (22) außerhalb der konvergenten Oberfläche plastisch deformiert, um das Fenster in der Ummantelung zu halten und zu sichern.

Fig. 2.

Fig. 3.

Fig. 1.